# EUROPÄISCHE PATENTSCHRIFT

(11) **EP 0 759 420 B1**
(45) Veröffentlichungstag und Bekanntmachung des Hinweises auf die Patenterteilung: **08.11.2000**
(21) Anmeldenummer: 96113142.2
(22) Anmeldetag: 16.08.1996
(51) Int. Cl.: C07C 51/14, C07C 53/122

(54) **Verfahren zur Herstellung von Carbonsäuren durch Carbonylierung von Olefinen**
Process for preparing carboxylic acids by carbonylation of olefins
Procédé de préparation d'acides carboxyliques par carbonylation d'oléfines

(30) Priorität: 23.08.1995 DE 19530992
(43) Veröffentlichungstag der Anmeldung: 26.02.1997
(73) Patentinhaber: BASF Aktiengesellschaft, 67063 Ludwigshafen (DE)
(72) Erfinder: Schäfer, Martin, Dr., 67063 Ludwigshafen (DE); Höhn, Arthur, Dr., 67281 Kirchheim (DE); Lippert, Ferdinand, Dr., 67098 Bad Dürkheim (DE)

(56) Entgegenhaltungen:
- DE-A- 2 404 955
- US-A- 4 588 834

## Beschreibung

Die vorliegende Erfindung betrifft ein Verfahren zur Herstellung von Carbonsäuren durch Umsetzung eines Olefins mit Kohlenmonoxid in Gegenwart von Wasser und eines halogenfreien Katalysatorsystems bei Temperaturen von 50-150°C und Drücken von 30-150 bar, einer Mischung aus Rhodium oder einer Rhodium-Verbindung und mindestens einer stickstoffhaltigen heterocyclischen Verbindung, in Abwesenheit eines Aktivkohlefestbetts.

Weissermel et al. beschreiben in Industrielle Organische Chemie, 1978, 2. Auflage, Verlag Chemie, S. 132 die Carbonylierung von Olefinen nach dem Reppe-Verfahren, beispielsweise die Herstellung von Propionsäure aus Ethylen, Kohlenmonoxid und Wasser in Gegenwart von Katalysatoren. Als Katalysator wird Nickelpropionat eingesetzt, das sich unter Reaktionsbedingungen zu Nickelcarbonyl umsetzt. Ein hoher Umsatz des Kohlenmonoxids wird nur bei hohen Drücken (200 bis 240 bar) erzielt. Diese Reaktionsbedingungen erfordern einen hohen technischen Aufwand beim Bau geeigneter Reaktoren sowie - bedingt durch die Korrosivität des Produktes unter den Reaktionsbedingungen - besondere und teure Werkstoffe.

Carbonylierungen von Olefinen können mit Edelmetallkatalysatoren bei Drücken von ca. 100 bar durchgeführt werden. So lehrt die EP-A-495 547 Katalysatoren, die aus einer Palladiumquelle und zweizähnigen Phosphinliganden bestehen. Solche Katalysatoren werden aber häufig nach kurzer Reaktionsdauer durch Abscheidung metallischen Palladiums desaktiviert; insbesondere sind die eingesetzten Phosphinliganden unter den gewünschten Reaktionsbedingungen nicht thermostabil.

Die DE-A-21 01 909 betrifft einen Rhodium-Halogenid-Carbonyl-Katalysator, der ohne Zusatz eines weiteren Halogenidpromoters Olefine in Gegenwart von Wasser und CO in Carbonsäuren überführt. Gute Ausbeuten und Selektivitäten an einer Carbonsäure wie beispielsweise Propionsäure werden mit diesem System nur dann erzielt, wenn bei CO/Olefin-Verhältnissen größer als 2/1 gearbeitet wird. So lassen sich beispielsweise bei einem molaren Verhältnis CO/Ethen von 8/1 mit [RhCl(CO)₂]₂ als Katalysator Reaktionsraten zwischen 233 und 520 g Propionsäure/h/g Rh erreichen. Bei einem Verhältnis CO/Ethen von 1/1 beträgt die Reaktionsrate dagegen nur 105 g Propionsäure/h/g Rhodium. Wird ein halogenfreier Rhodium-Katalysator wie Rh₄(CO)₁₂ eingesetzt, sinkt die Reaktionsrate auf 51 g Propionsäure/h/g Rhodium.

Die DE-A-22 63 442 (US-A-3 816 490) lehrt ein Verfahren zur Herstellung von Carbonsäuren und Carbonsäureanhydriden aus Olefinen mit einem halogenfreien Rhodium- bzw. Iridium-Katalysator in Gegenwart von Phenol- oder Thiophenol-Derivaten bzw. fluorierten Carbonsäuren, Thiocarbonsäuren oder Sulfonsäuren. Die katalytische Aktivität ist jedoch vergleichsweise gering.

Es stellte sich die Aufgabe, ein Verfahren zur Carbonylierung von Olefinen bereitzustellen, das die oben angegebenen Nachteile vermeidet.

Demgemäß wurde ein neues und verbessertes Verfahren zur Herstellung von Carbonsäuren aus Olefinen und Kohlenmonoxid in Gegenwart von Wasser und eines halogenfreien Katalysatorsystems bei Temperaturen von 30 bis 200°C und Drücken von 30 bis 200 bar, gefunden, welches dadurch gekennzeichnet ist, daß man als Katalysatorsystem eine Mischung aus Rhodium oder einer Rhodium-Verbindung und mindestens eine stickstoffhaltige heterocyclische Verbindung einsetzt.

Als Ausgangsstoffe für das erfindungsgemäße Verfahren kommen aliphatische und cycloaliphatische Alkene mit vorzugsweise 2 bis 20, besonders bevorzugt 2 bis 7 Kohlenstoffatomen in Betracht. Beispielhaft sind Ethylen, Propylen, iso-Buten, 1-Buten, 2-Buten und die Isomeren des Pentens und Hexens, Octen sowie Cyclopenten zu nennen, von denen Ethylen bevorzugt ist. Diese Olefine werden zur Herstellung von Carbonsäuren mit Wasser und CO umgesetzt.

Als stickstoffhaltige heterocyclische Verbindung eignen sich für das erfindungsgemäße Verfahren beispielsweise Derivate des Pyridins, Chinolins, Isochinolins, Pyrimidins, Pyridazins, Pyrazins, Pyrazols, Imidazols, Thiazols und Oxazols, von denen die Derivate des Pyridins, Chinolins und Isochinolins bevorzugt, die Derivate des Pyridins besonders bevorzugt sind.

Als Derivate des Pyridins können neben Pyridin selbst beispielweise durch Alkyl oder Aryl ein bis dreifach substituierte Verbindungen wie Picolin, Lutidin oder Collidin eingesetzt werden. Besonders geeignet sind Pyridin sowie einfach substituierte Derivate wie 3-Picolin, 4-Picolin, 4-t-Butylpyridin, 4-Benzylpyridin, 4-(Phenylpropyl)pyridin, 4-(Pyridylpropyl)pyridin, 4-Phenylpyridin sowie 2-(Pyridyl)ethansulfonsäure und deren Salze.

Das Pyridinderivat kann auch an einen organischen oder anorganischen Träger gebunden sein. Besonders geeignet sind Polymere oder Copolymere des 4-Vinylpyridins, die in Abhängigkeit von ihrer Molmasse und ihrem Vernetzungsgrad im Reaktionsmedium löslich oder unlöslich sein können. Im Fall unlöslicher Polymere kann sich der Rhodium-Katalysator nach der Carbonylierungsreaktion auf dem Träger abscheiden und durch Abfiltrieren aus dem Reaktionsgemisch entfernt werden.

Kohlenmonoxid kann in reiner Form oder verdünnt mit inerten Gasen wie Stickstoff oder Argon eingesetzt werden.

Die Molverhältnisse der Ausgangsverbindungen Olefin und Wasser können in weiten Grenzen variieren, jedoch wird in der Regel mindestens eine äquimolare Menge an Wasser eingesetzt. Es kann ein großer Wasserüberschuß gewählt werden, z.B. 2 bis 10 mol Wasser pro Olefin.

Auch das Molverhältnis von Olefin zu Kohlenmonoxid kann stark varriert werden, z.B. zwischen 5:1 und 1:5 mol Olefin pro mol Kohlenmonoxid. Die Herstellung von Propionsäure wird in der Regel bevorzugt im Molverhältnis von CO zu Ethen von 0,9:1 bis 2:1, besonders bevorzugt 1:1 durchgeführt.

Als Katalysator können im erfindungsgemäßen Verfahren halogenfreie Rhodium-Verbindungen sowie mindestens eine stickstoffhaltige heterocyclische Verbindung eingesetzt werden. Damit sich die aktiven Komponenten des Rhodiums bilden können, werden dem Reaktionsgemisch zweckmäßigerweise lösliche Rhodium-Verbindungen zugesetzt, wie beispielsweise Acetate, Propionate, Acetylacetonate, Oxide, Hydroxide und Carbonate. Bei der Verwendung halogenfreier Rhodium(III)-Verbindung als Vorstufen kann gegebenenfalls die Bildung des aktiven Katalysators durch Zudosierung von H₂ zum Reaktionsgemisch beschleunigt werden. Die Aktivierung (Vorcarbonylierung) des Katalysators kann auch in einem separaten Reaktionsraum durch Umsetzung mit CO und Wasser bzw. mit CO und H₂ bei Temperaturen zwischen 50 und 150°C und Drücken zwischen 50 und 150 bar erfolgen. Weiterhin kommen Carbonyl-Verbindungen in Betracht wie Rh(acac)(CO)₂, Rh₄(CO)₁₂, Rh₆(CO)₁₆ oder Salze des Anions [Rh₁₂(CO)₃₀]²⁻ sowie durch Donorliganden (z.B. N-Basen) oder Olefine stabilisierte Rhodium-Verbindungen.

Der Gehalt der Reaktionslösung an Rhodium beträgt im allgemeinen 0,001 bis 1 Gew.-%, bevorzugt 0,005 bis 0,5 Gew.-%, besonders bevorzugt 0,01 bis 0,3 Gew.-% berechnet als Metall.

Als weitere Katalysatorkomponenete dient mindestens eine, also 1, 2, 3, 4, 5, 6, 7, 8 oder mehrere, bevorzugt 1, 2, 3, 4 oder 5, besonders bevorzugt 1, 2 oder 3, insbesondere 1 oder 2 stickstoffhaltige heterocyclische Verbindungen. Bevorzugt sind Pyridin und Pyridinderivate wie Picolin und Lutidin. Der Gehalt des Reaktionsgemischs an diesen Basen beträgt 1 bis 50 Gew.-%, bevorzugt 10 bis 30 Gew.-%.

Das Molverhältnis der stickstoffhaltigen heterocyclischen Verbindung zum Rhodium liegt im allgemeinen bei 10:1 bis 10000:1, bevorzugt bei 50:1 bis 1500:1.

Die Umsetzung kann mit oder ohne Lösungsmittel durchgeführt werden.

Wird kein zusätzliches Lösungsmittel eingesetzt, so ist bevorzugt, die Umsetzung in der 10 bis 80 gew.-%igen, bevorzugt 20 bis 70 gew.-%igen wässrigen Carbonsäure durchzuführen, die hergestellt wird. Bei der Herstellung von Propionsäure ist die Verwendung von wässriger Propionsäure als Lösungsmittel bevorzugt.

Als Lösungsmittel kommen neben wässrigen Carbonsäuren aprotisch polare Lösungsmittel wie Aceton, N-Methylpyrrolidon und Ether wie Diethylether, Dioctylether, Diethoxyethan, Dioxan, Diethylenglykoldiethylether, Diethylenglykoldioctylether sowie hochsiedende aliphatische Kohlenwasserstoffe und aromatische Kohlenwasserstoffe wie Toluol in Betracht. In Abhängigkeit von Art und Menge des zugesetzten Lösungsmittels kann das Reaktionsgemisch ein- oder zweiphasig vorliegen.

Bevorzugt ist die Verwendung von Ethern der allgemeinen Formel (I) 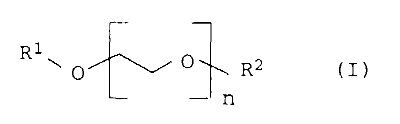 in der
- R¹ und R²: Wasserstoff, C₁- bis C₂₀-Alkyl, Aryl, 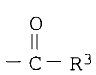 oder R¹ und R² gemeinsam eine C₀- bis C₂₀-Alkylenkette
- R³: Wasserstoff, C₁- bis C₂₀-Alkyl oder Aryl und
- n: 0 bis 30
bedeuten, mit der Maßgabe, daß R¹ und R² nicht 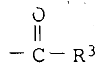 bedeuten, wenn n für 0 oder 1 steht sowie R¹ und R² nicht Wasserstoff bedeuten, wenn n für 0 steht, als Lösungsmittel, wobei die Verbindungen mit R¹, R² = C₁- bis C₂₀-Alkyl, insbesondere Ethyl, Propyl, Butyl, Pentyl, Hexyl, Heptyl, Octyl, Decyl, Ethylhexyl besonders bevorzugt sind. In (I) eingeschlossen sind auch zyklische Ether wie Dioxan, wenn die Gruppen R¹ und R² durch mindestens eine Bindung verknüpft sind.

Der Gehalt des Reaktionsgemischs an einem zusätzlichen Lösungsmittel kann in einem weiten Bereich variiert werden. Er beträgt im allgemeinen 20 bis 80 Gew.-%, bevorzugt 30 bis 60 Gew.-%.

Die Verwendung eines zusätzlichen Lösungsmittels fördert die Löslichkeit des Olefins, des Kohlenmonoxids und des aktiven Katalysators im Reaktionsgemisch. So ermöglicht beispielsweise bei der Carbonylierung von Ethen, die Verwendung der beschriebenen Ether als Lösungsmitteln die Synthese von Propionsäure mit hoher Ausbeute und Selektivität bei milderen Reaktionsbedingungen als mit Propionsäure/Wasser als Lösungsmittel.

Die Reaktion wird in der Regel bei 30 bis 200°C, vorzugsweise bei 50 bis 150°C und Drücken von 30 bis 250 bar durchgeführt. Höhere Drücke und höhere Temperaturen führen in der Regel zur vermehrten Bildung von Nebenprodukten wie Ketone, Alkane, Aldehyde. Bei der Carbonylierung endständiger Olefine fördern höhere Temperaturen die Doppelbindungsisomerisierung.

Bei der Synthese von Propionsäure wird die Reaktion vorzugsweise bei 50 bis 150 bar, bei der Synthese höherer Carbonsäuren wie Nonansäure vorzugsweise bei 100 bis 250 bar durchgeführt.

Die Ausgangsverbindungen Olefin, Wasser und das Katalysatorsystem können vor der Umsetzung gegebenenfalls in einem Lösungsmittel in einem Reaktor vermischt werden. Sie können dann auf die Reaktionstemperatur erhitzt werden, wobei der Reaktionsdruck durch Aufpressen von Kohlenmonoxid oder bei Verwendung kurzkettiger Olefine durch Aufpressen eines Gemisches aus diesem Olefin und Kohlenmonoxid eingestellt wird.

In der Regel ist die Umsetzung nach 0,5 bis 3 h beendet. Sie kann kontinuierlich oder diskontinuierlich in Reaktoren wie Kesseln, Blasensäulen, Rohrreaktoren oder Umlaufreaktoren ausgeführt werden.

Zur Isolierung der Verfahrensprodukte wird der Reaktionsaustrag in einer bevorzugten Ausführungsform entspannt. Die Flüssigphase des Reaktionsaustrags, die neben dem Verfahrensprodukt löslichen oder suspendierten Katalysator enthält, wird destillativ aufgearbeitet, wobei das Verfahrensprodukt, gegebenenfalls nach einer anschließenden Feindestillation, isoliert wird. Der katalysatorhaltige Destillationssumpf wird in die Reaktion zurückgeführt. Ebenso können gegebenenfalls vor der Destillation abgetrennte Katalysatorbestandteile sowie als Leichtsieder oder als Seitenstrom einer Destillation abgetrennte flüchtige Katalysatorbestandteile nach entsprechender Aufarbeitung zurückgeführt werden.

Der im flüssigen Reaktionsaustrag vorliegende aktive Katalysator wird vor der destillativen Aufarbeitung gegebenenfalls durch Reaktion mit Sauerstoff oder Luft bei 50 bis 150°C und 0,5 bis 10 bar inaktiviert.

Das erfindungsgemäße Verfahren erlaubt die Herstellung der Verfahrensprodukte unter moderaten Reaktionsbedingungen in hoher Raum-Zeit-Ausbeute mit hoher Selektivität.

### Beispiele

Diskontinuierliche Versuche zur Herstellung von Propionsäure

### Beispiele 1 bis 20

In einem Autoklaven wurden Rh(acac)(CO)₂ und das Pyridinderivat in einem Gemisch aus Propionsäure und Wasser als Lösungsmittel vorgelegt. Mit einem Gemisch aus 50 Vol.-% Ethen und 50 Vol.-% CO wurde ein Druck von 30 bar eingestellt und das Gemisch auf die entsprechende Reaktionstemperatur gebracht. Danach wurde der gewünschte Reaktionsdruck durch Aufpressen des CO/Ethen-Gemisches eingestellt und durch Nachdrücken (alle 15 Minuten) gehalten. Nach 1 h wurde entspannt und der Reaktionsaustrag titrimetrisch und gaschromatographisch analysiert. Die Ergebnisse sind in Tabelle 1 zusammengefaßt.

### Verwendete Abkürzungen:

VWZ = Verweilzeit, Versuchsdauer, RZA = Raum-Zeit-Ausbeute, Sel. = Selektivität bez. Ethen, PS = Propionsäure, PA = Propionaldehyd, DEK = Diethylketon, 4-OHS = 4-Oxohexansäure, py = Pyridin, 3-Mepy = 3-Methylpyridin, 4-Mepy = 4-Methylpyridin, 4-tBupy = 4-tertiär-Butylpyridin, 4-CH₂Phpy = 4-Benzylpyridin, 4-Phpy = 4-Phenylpyridin, 4-(CH₂)₃Phpy = (3-Phenyl)propylpyridin, 4,4'-py(CH₂)₃py = 4,4'-Trimethylendipyridin, 2,4,6-Me₃py = 2,4,6-Trimethylpyridin

### Vergleichsbeispiele 21 bis 24

In einem Autoklaven wurde Rh(acac)(CO)₂ in einem Gemisch aus Propionsäure und Wasser als Lösungsmittel vorgelegt. Bei den Beispielen 22 und 23 wurde Pyridin zugefügt. Mit einem Gemisch aus 50 Vol.-% Ethen und 50 Vol.-% CO wurde ein Druck von 30 bar eingestellt und das Gemisch auf die entsprechende Reaktionstemperatur gebracht. Danach wurde der gewünschte Reaktionsdruck durch Aufpressen des CO/Ethen-Gemisches eingestellt und durch Nachdrücken (alle 15 Minuten) gehalten. Nach 1 h wurde entspannt und der Reaktionsaustrag titrimetrisch und gaschromatographisch analysiert. Die Ergebnisse sind in Tabelle 2 zusammengefaßt.

### Beispiel 25

In einem Autoklaven wurde 0,08 g Rh₂(OAc)₄ und 10 g Pyridin in einem Gemisch aus Propionsäure und Wasser als Lösungsmittel vorgelegt. Mit einem Gemisch aus 50 Vol.-% Ethen und 50 Vol.-% CO wurde ein Druck von 30 bar eingestellt und das Gemisch auf 100°C aufgeheizt. Danach wurde der gewünschte Reaktionsdruck durch Aufpressen des CO/Ethen-Gemisches eingestellt und durch Nachdrücken (alle 15 Minuten) gehalten. Nach 1 h wurde entspannt und der Reaktionsaustrag titrimetrisch und gaschromatographisch analysiert. Die Ergebnisse sind in Tabelle 3 zusammengefaßt.

### Beispiel 26

Wie Beispiel 25 mit 0,11 g Na₂[Rh₁₂(CO)₃₀] und 0,2 g NBu₄OH als Katalysator. Die Ergebnisse sind in Tabelle 3 zusammengefaßt.

### Beispiel 27

In einem Autoklaven wurde 0,22 g Rh(OAc)₃ und 10 g Pyridin in einem Gemisch aus Propionsäure und Wasser als Lösungsmittel vorgelegt. Anschließend wird das Gemisch auf 100°C aufgeheizt. Danach wurde der gewünschte Reaktionsdruck durch Aufpressen des CO/ELhen-Gemisches eingestellt und durch Nachdrücken (alle 15 Minuten) gehalten. Nach 1 h wurde entspannt und der Reaktionsaustrag titrimetrisch und gaschromatographisch analysiert. Die Ergebnisse sind in Tabelle 3 zusammengefaßt.

### Beispiel 28

In einem Autoklaven wurde 0,22 g Rh(OAc)₃ und 10 g Pyridin in einem Gemisch aus Propionsäure und Wasser als Lösungsmittel vorgelegt. Mit einem 1/1-Gemisch aus CO/H₂ wird ein Vordruck von 10 bar eingestellt. Anschließend wird das Gemisch auf 100°C aufgeheizt. Danach wurde der gewünschte Reaktionsdruck durch Aufpressen des CO/Ethen-Gemisches eingestellt und durch Nachdrücken (alle 15 Minuten) gehalten. Nach 1 h wurde entspannt und der Reaktionsaustrag titrimetrisch und gaschromatographisch analysiert. Die Ergebnisse sind in Tabelle 3 zusammengefaßt.

### Beispiele 29 bis 39

In einem Autoklaven wurden 0,22 g Rh(acac)(CO)₂ und 10 g des Pyridinderivats in einem Gemisch aus 50 g Ether und 40 g Wasser vorgelegt. Mit einem Gemisch aus 50 Vol.-% Ethen und 50 Vol.-% CO wurde ein Druck von 30 bar (bei Versuch 36 wurden 30 bar CO als Vordruck aufgepresst) eingestellt und das Gemisch auf die entsprechende Reaktionstemperatur gebracht. Danach wurde der gewünschte Reaktionsdruck durch Aufpressen des CO/Ethen-Gemisches eingestellt und durch Nachdrücken (alle 15 Minuten) gehalten. Nach 1 h wurde entspannt und der Reaktionsaustrag titrimetrisch und gaschromatographisch analysiert. Die Ergebnisse sind in Tabelle 4 zusammengefaßt.

### Beispiel 40

a) In einem Autoklaven wurden 0,22 g Rh(acac)(CO)₂ und 10 g eines kommerziell erhältlichen 4-Vinylpyridinpolymers (Fa. Aldrich, 2% vernetzt) in einem Gemisch aus 50 g Propionsäure und 40 g Wasser vorgelegt. Mit einem Gemisch aus 50 Vol.-% Ethen und 50 Vol.-% CO wurde ein Druck von 30 bar (bei Versuch 36 wurden 30 bar CO als Vordruck aufgepresst) eingestellt und das Gemisch auf 100°C aufgeheizt. Danach wurde der Reaktionsdruck von 100 bar durch Aufpressen des CO/Ethen-Gemisches eingestellt und durch Nachdrücken (alle 15 Minuten) gehalten. Nach 1 h wurde entspannt und der Reaktionsaustrag titrimetrisch und gaschromatographisch analysiert. Propionsäure entstand mit der RZA von 214 g/l/h und der Selektivität von 90 %.
b) Das im Reaktionsaustrag von a) enthaltene Polymer wurde abfiltriert, getrocknet und ohne Zusatz von Rhodium wie unter a) beschrieben mit CO/Ethen umgesetzt. Propionsäure entstand mit der RZA von 208 g/l/h und der Selektivität von 91 %.

Dieser Versuch verdeutlicht, daß der Rh-Katalysator nach der Reaktion auf dem Pyridin-Polymer abgeschieden, vom Reaktionsaustrag abgetrennt, und mit dem Polymer in die Reaktion zurückgeführt werden kann.

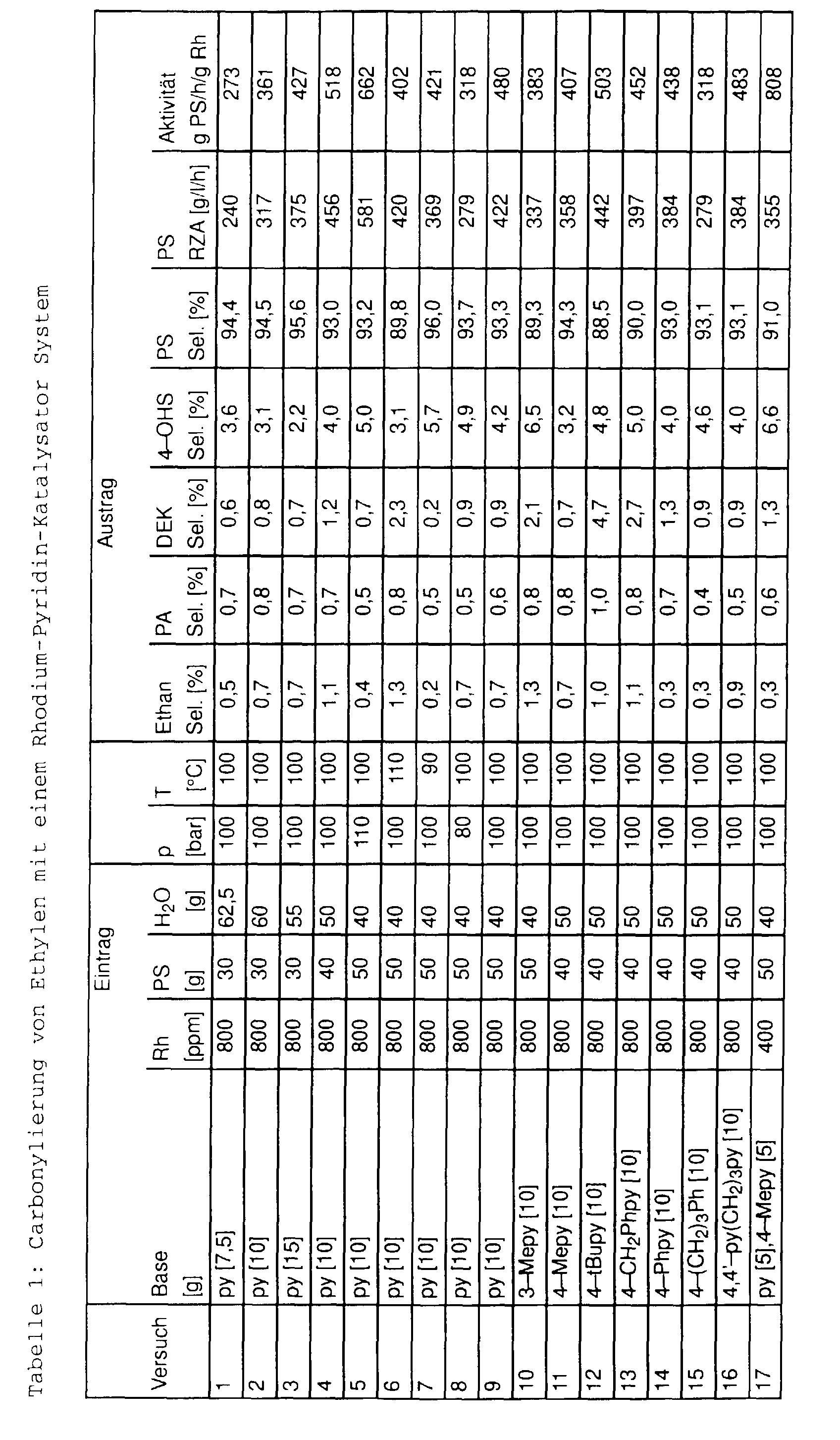

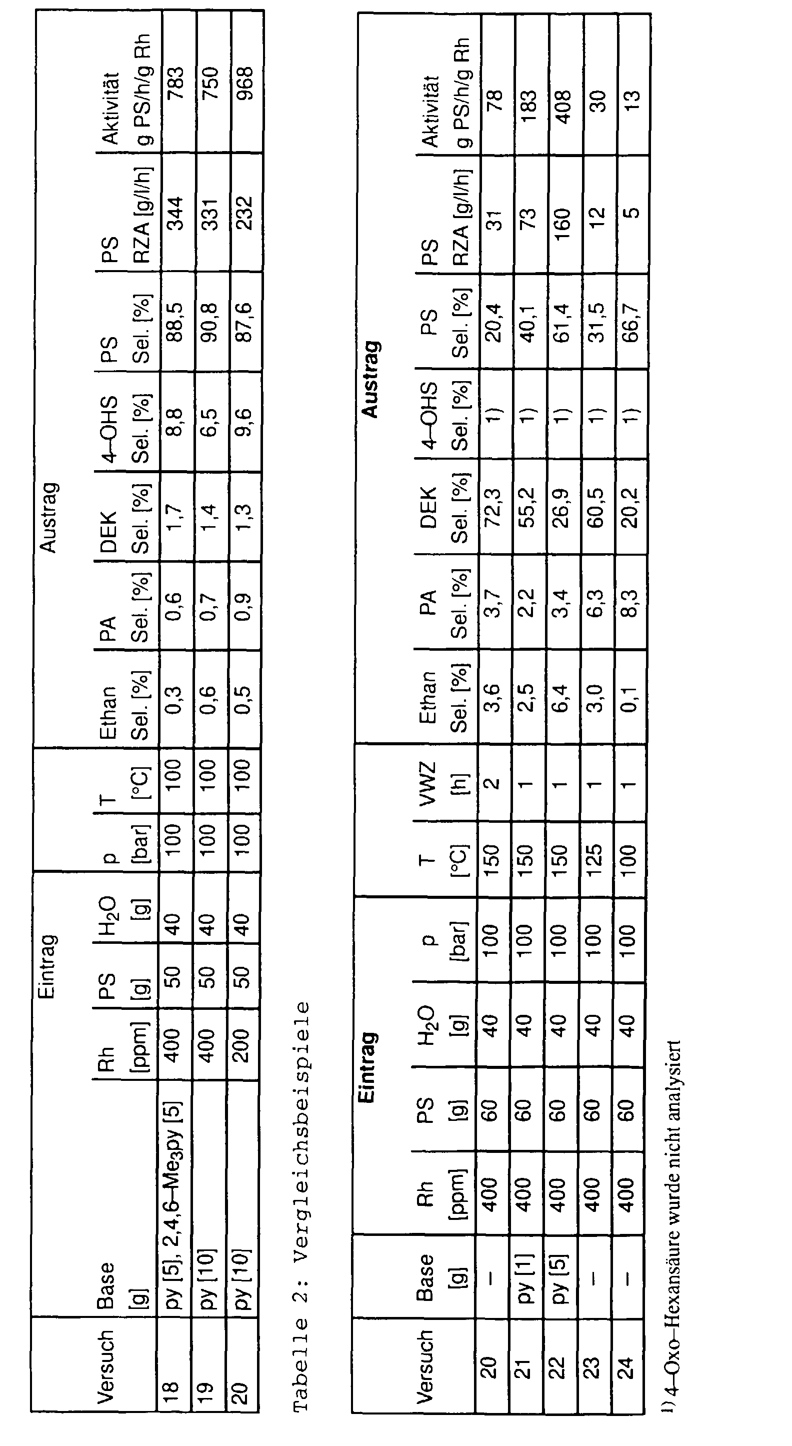

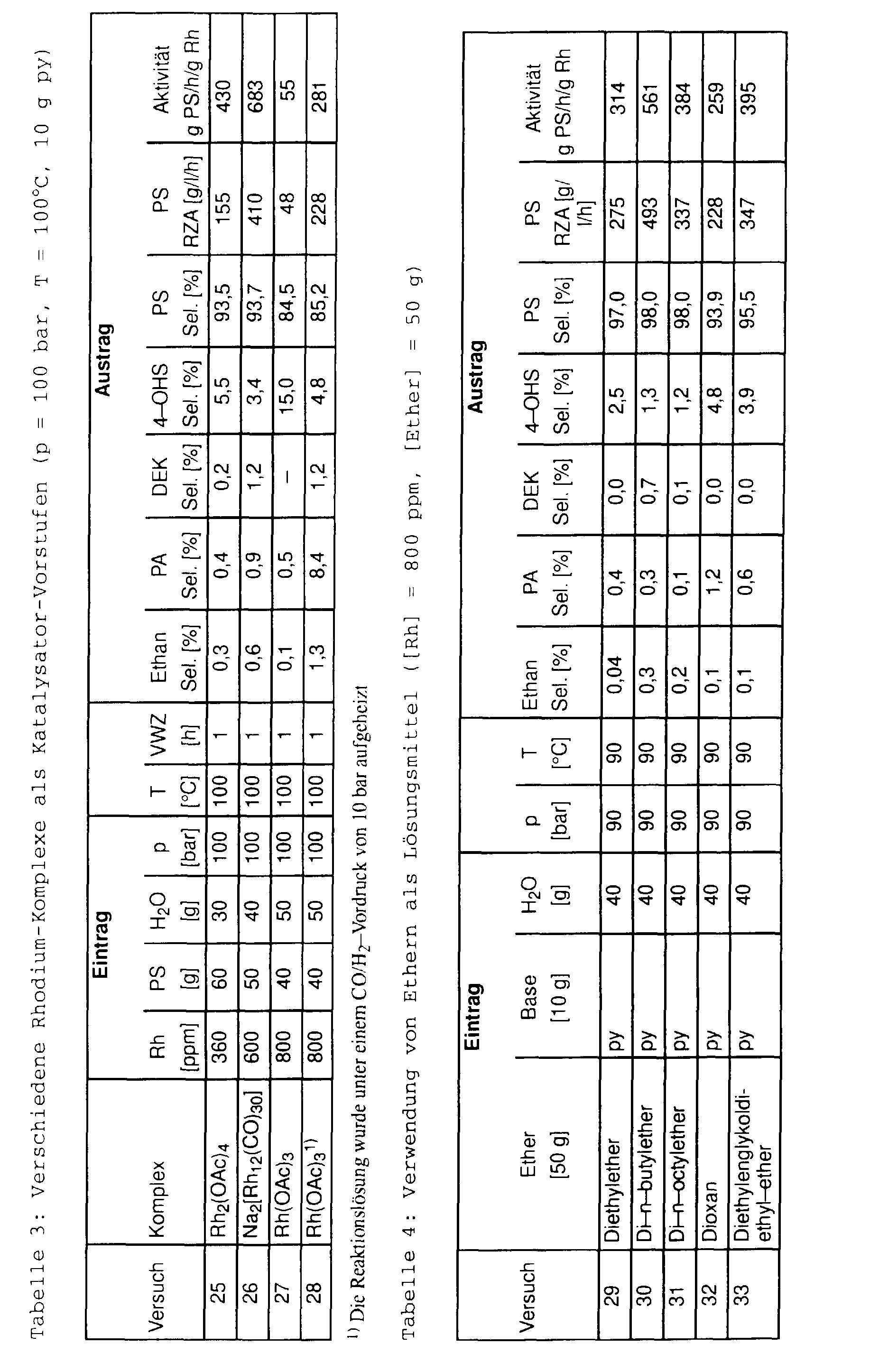

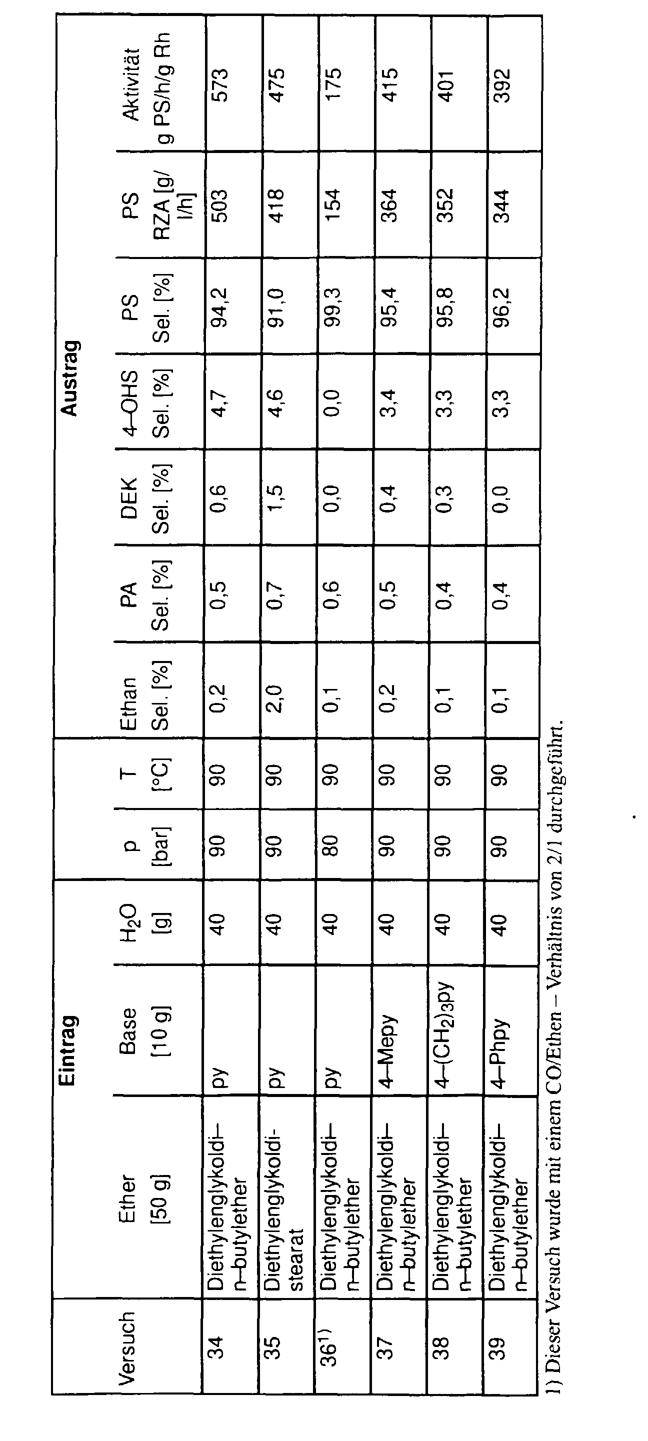

### Versuch 41

In einem Autoklaven wurden 0,1 g Rh(acac)(CO)₂, 20 g Pyridin und 10 g 1-Octen in einem Gemisch aus 60 g Propionsäure und 10 g Wasser als Lösungsmittel vorgelegt. Mit CO wurde ein Druck von 30 bar eingestellt und das Gemisch auf 100°C aufgeheizt. Danach wurde ein Reaktionsdruck von 100 bar durch Aufpressen von CO eingestellt und durch Nachdrücken (alle 15 Minuten) gehalten. Nach 1 h wurde entspannt und der Reaktionsaustrag titrimetrisch und gaschromatographisch analysiert. Nonansäure (n/iso = 2,6:1) entstand mit einer RZA von 46 g/l/h (Sel. > 95%). Nonanal (n/iso) konnte gaschromatographisch in Spuren nachgewiesen werden. Hydrierungsprodukte wie Octan oder Nonanol wurden nicht gefunden.

### Versuch 42

In einem Autoklaven wurden 0,55 g Rh(acac)(CO)₂, 10 g 4-Methylpyridin und 20 g 1-Octen in einem Gemisch aus 50 g Diethylenglykoldi-n-butylether und 10 g Wasser vorgelegt. Mit CO wurde ein Druck von 30 bar eingestellt und das Gemisch auf 90°C aufgeheizt. Danach wurde ein Reaktionsdruck von 150 bar durch Aufpressen von CO eingestellt und durch Nachdrücken (alle 15 Minuten) gehalten. Nach 1 h wurde entspannt und der Reaktionsaustrag titrimetrisch und gaschromatographisch analysiert. Nonansäure (n/iso = 3,8:1) entstand mit einer RZA von 135 g/l/h (Sel. > 95%). Nonanal (n/iso) konnte gaschromatographisch in Spuren nachgewiesen werden. Hydrierungsprodukte wie Octan oder Nonanol wurden nicht gefunden.

### Kontinuierliche Versuche

### Beispiele 43 bis 48

Eine Mischung aus Propionsäure, Wasser, Pyridin und Rh(acac)(CO)₂ wurde kontinuierlich bei 100°C und 100 bar mit CO und Ethen umgesetzt.Nach Verweilzeiten zwischen 0,5 und 2,1 Stunden wurde kontinuierlich Reaktionsgemisch entnommen und analysiert. Die Ergebnisse dieser Versuche sind in Tabelle 5 zusammengefasst.

### Beispiel 49

Eine Mischung aus Diethylenglykoldi-n-butylether, Wasser, Pyridin und Rh(acac)(CO)₂ wurde kontinuierlich bei 100°C und 100 bar mit CO und Ethen umgesetzt. Nach einer Verweilzeit von 2,7 Stunden wurde kontinuierlich Reaktionsgemisch entnommen und analysiert. Das Ergebnis dieses Versuchs ist in Tabelle 5 zusammengefasst.

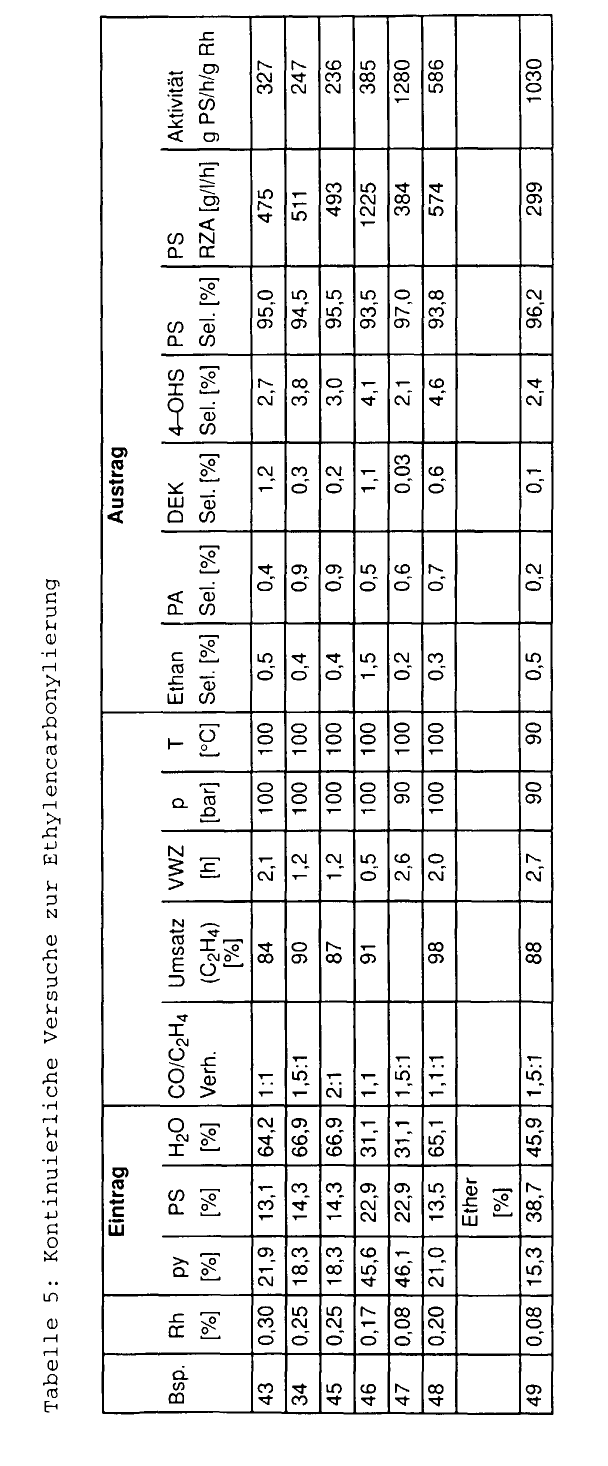

### Kontinuierliche Versuche zur Katalysatorrückführung

### Beispiel 50

In einem Carbonylierungsreaktor wurde bei 100°C und 100 bar mit Rhodium und Pyridin als Katalysatoren in wässriger Propionsäure Kohlenmonoxid mit Ethen (CO/C₂H4 = 1,1/1) mit Wasser zu Propionsäure kontinuierlich umgesetzt. Nach einer Verweilzeit von 2 Stunden wurde am Reaktorkopf kontinuierlich ein Reaktionsgemisch mit der Zusammensetzung 68% PS, 18% H₂O, 11% py, 3,3 % 4-OHS, 0,2 % PA, 0,02 % DEK, 0,23 % Rh entnommen. Zur Passivierung des Rhodium-Katalysators wurde der flüssige Reaktionsaustrag in einem Oxidationsreaktor (VWZ = 0,5 Stunden) bei 100°C und 3 bar mit Luft umgesetzt. Anschließend wurden in einem Sambayverdampfer bei 150°C und 0,5 bar das Produkt und Nebenprodukte sowie Wasser und ein Teil des Pyridins abgetrennt. Der Rhodium-haltige Sumpf, der neben 4-Oxo-Hexansäure auch Pyridin und Propionsäure enthält, wurde mit CO behandelt und in den Carbonylierungsreaktor zurückgeführt. Das über den Sambaykopf ausgetragene Pyridin wurde kontinuierlich ergänzt und zusammen mit Frischwasser und Propionsäure in den Carbonylierungsreaktor zudosiert. Der Versuch wurde über einen Zeitraum von 70 Stunden durchgeführt und lieferte Propionsäure mit der RZA von 350 g/l/h und einer Sel. von 95 % (Sel. (4-OHS) = 3 %, Sel. (DEK) = 0,1 %, Sel. (PA) = 0,9 %, Sel. (C₂H₆) = 0,2 %). Der Ethenumsatz betrug 99 %.

Aus dem Sambaykondensat (Zusammensetzung: 67 % PS, 24 % H₂O, 6,5 % py, 1,4 % 4-OHS, 0,4 % PA, 0,05 % DEK) kann destillativ Propionsäure mit einer Reinheit > = 98,5 % gewonnen werden. Pyridin fällt als Hochsiederazeotrop mit Propionsäure an und kann in die Reaktion zurückgeführt werden.

## Patentansprüche

1. Verfahren zur Herstellung von Carbonsäuren aus Olefinen und Kohlenmonoxid in Gegenwart von Wasser und eines halogenfreien Katalysatorsystems bei Temperaturen von 50 bis 150°C und Drücken von 30 bis 150 bar, dadurch gekennzeichnet, daß man als Katalysatorsystem eine Mischung aus Rhodium oder einer Rhodium-Verbindung und mindestens eine stickstoffhaltige heterocyclische Verbindung in Abwesenheit eines Aktivkohlefestbetts einsetzt.

2. Verfahren zur Herstellung von Carbonsäuren aus Olefinen und Kohlenmonoxid nach Anspruch 1, dadurch gekennzeichnet, daß man die stickstoffhaltige heterocyclische Verbindung und das Rhodium oder eine Rhodium-Verbindung im Molverhältnis von 10000:1 bis 10:1 einsetzt.

3. Verfahren zur Herstellung von Carbonsäuren aus Olefinen und Kohlenmonoxid nach den Ansprüchen 1 bis 2, dadurch gekennzeichnet, daß der Gehalt der Reaktionslösung an der stickstoffhaltigen heterocyclischen Verbindung 1 bis 30 Gew.-% beträgt.

4. Verfahren zur Herstellung von Carbonsäuren aus Olefinen und Kohlenmonoxid nach den Ansprüchen 1 bis 3, dadurch gekennzeichnet, daß man als stickstoffhaltige heterocyclische Verbindung Pyridin, dessen Derivate oder Gemische aus diesen einsetzt.

5. Verfahren zur Herstellung von Carbonsäuren aus Olefinen und Kohlenmonoxid nach den Ansprüchen 1 bis 4, dadurch gekennzeichnet, daß man die Umsetzung in einem aprotisch polaren Lösungsmittel durchführt.

6. Verfahren zur Herstellung von Carbonsäuren aus Olefinen und Kohlenmonoxid nach den Ansprüchen 1 bis 5, dadurch gekennzeichnet, daß man als aprotisch polare Lösungsmittel Ether der allgemeinen Formel (I) 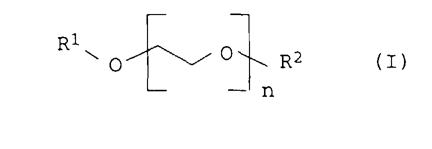 in der
R¹ und R² Wasserstoff, C₁- bis C₂₀-Alkyl, Aryl, 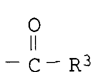 oder R¹ und R² gemeinsam eine C₀- bis C₂₀-Alkylenkette
R³ Wasserstoff, C₁- bis C₂₀-Alkyl oder Aryl und
n 0 bis 30
bedeuten, mit der Maßgabe, daß R¹ und R² nicht 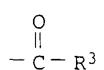 bedeuten, wenn n für 0 oder 1 steht sowie R¹ und R² nicht Wasserstoff bedeuten, wenn n für 0 steht, einsetzt.

7. Verfahren zur Herstellung von Carbonsäuren aus Olefinen und Kohlenmonoxid nach den Ansprüchen 1 bis 4, dadurch gekennzeichnet, daß man die Umsetzung in 10 bis 90 Gew.-% wäßriger Carbonsäure als Lösungsmittel durchführt.

8. Verfahren zur Herstellung von Carbonsäuren aus Olefinen und Kohlenmonoxid nach den Ansprüchen 1 bis 6, dadurch gekennzeichnet, daß man Kohlenmonoxid und das Olefin im Molverhältnis von 0,9:1 bis 20:1 einsetzt.

9. Verfahren zur Herstellung von Carbonsäuren aus Olefinen und Kohlenmonoxid nach den Ansprüchen 1 bis 7, dadurch gekennzeichnet, daß man als Olefin Ethylen einsetzt.

10. Verfahren zur Herstellung von Carbonsäuren aus Olefinen und Kohlenmonoxid nach den Ansprüchen 1 bis 8, dadurch gekennzeichnet, daß man Kohlenmonoxid und Ethen im Molverhältnis von 0,9:1 bis 1,2:1 einsetzt.

11. Verfahren zur Herstellung von Carbonsäuren aus Olefinen und Kohlenmonoxid nach den Ansprüchen 1 bis 9, dadurch gekennzeichnet, daß man die Umsetzung in Abwesenheit von Halogenwasserstoff oder eines Halogen-Promoters durchführt.

## Claims

1. A process for preparing carboxylic acids from olefins and carbon monoxide in the presence of water and a halogen-free catalyst system at from 50 to 150°C and pressures of from 30 to 150 bar, wherein the catalyst system used is a mixture of rhodium or a rhodium compound and at least one nitrogen-containing heterocyclic compound in the absence of a fixed bed of activated carbon.

2. A process for preparing carboxylic acids from olefins and carbon monoxide as claimed in claim 1, wherein the nitrogen-containing heterocyclic compound and the rhodium or rhodium compound are used in a molar ratio of from 10000:1 to 10:1.

3. A process for preparing carboxylic acids from olefins and carbon monoxide as claimed in claim 1 or 2, wherein the content of the nitrogen-containing heterocyclic compound in the reaction solution is from 1 to 30 % by weight.

4. A process for preparing carboxylic acids from olefins and carbon monoxide as claimed in any of claims 1 to 3, wherein the nitrogen-containing heterocylic compound used is pyridine, a derivative thereof or a mixture of these.

5. A process for preparing carboxylic acids from olefins and carbon monoxide as claimed in any of claims 1 to 4, wherein the reaction is carried out in an aprotic polar solvent.

6. A process for preparing carboxylic acids from olefins and carbon monoxide as claimed in any of claims 1 to 5, wherein the aprotic polar solvent used is an ether of the general formula (I) 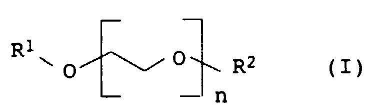 where
R¹ and R² are hydrogen, C₁-C₂₀-alkyl, aryl, 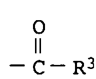 or R¹ and R² are together a C₁-C₂₀-alkylene chain
R³ is hydrogen, C₁-C₂₀-alkyl or aryl and
n is from 0 to 30,
with the proviso that R¹ and R² are not 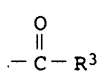 if n is 0 or 1 and R¹ and R² are not hydrogen if n is 0.

7. A process for preparing carboxylic acids from olefins and carbon monoxide as claimed in any of claims 1 to 4, wherein the reaction is carried out in a 10 - 90 % strength by weight aqueous solution of carboxylic acid as solvent.

8. A process for preparing carboxylic acids from olefins and carbon monoxide as claimed in any of claims 1 to 6, wherein carbon monoxide and the olefin are used in a molar ratio of from 0.9:1 to 20:1.

9. A process for preparing carboxylic acids from olefins and carbon monoxide as claimed in any of claims 1 to 7, wherein the olefin used is ethylene.

10. A process for preparing carboxylic acids from olefins and carbon monoxide as claimed in any of claims 1 to 8, wherein carbon monoxide and ethene are used in a molar ratio of from 0.9:1 to 1.2:1.

11. A process for preparing carboxylic acids from olefins and carbon monoxide as claimed in any of claims 1 to 9, wherein the reaction is carried out in the absence of hydrogen halide or a halogen promoter.

## Revendications

1. Procédé de préparation d'acides carboxyliques à partir d'oléfines et de monoxyde de carbone en présence d'eau et d'un système catalytique exempt d'halogènes, à des températures de 50 à 150°C et sous des pressions de 30 à 150 bar, caractérisé en ce que l'on utilise, en tant que système catalytique, un mélange de rhodium ou d'un composé du rhodium et d'au moins un composé hétérocyclique azoté en l'absence d'un lit fixe de charbon actif.

2. Procédé de préparation d'acides carboxyliques à partir d'oléfines et de monoxyde de carbone selon la revendication 1, caractérisé en ce que l'on utilise le composé hétérocyclique azoté et le rhodium ou le composé du rhodium dans un rapport en moles de 10 000 : 1 à 10 : 1.

3. Procédé de préparation d'acides carboxyliques à partir d'oléfines et de monoxyde de carbone selon les revendications 1 à 2, caractérisé en ce que la teneur de la solution réactionnelle en le composé hétérocyclique azoté s'élève à 1 à 30% en poids.

4. Procédé de préparation d'acides carboxyliques à partir d'oléfines et de monoxyde de carbone selon l'une quelconque des revendications 1 à 3, caractérisé en ce que l'on utilise, en tant que composé hétérocyclique azoté, de la pyridine, ses dérivés ou leurs mélanges.

5. Procédé de préparation d'acides carboxyliques à partir d'oléfines et de monoxyde de carbone selon l'une quelconque des revendications 1 à 4, caractérisé en ce que l'on effectue la réaction dans un solvant aprotique polaire.

6. Procédé de préparation d'acides carboxyliques à partir d'oléfines et de monoxyde de carbone selon l'une quelconque des revendications 1 à 5, caractérisé en ce que l'on utilise, en tant que solvant aprotique polaire un éther de formule générale (I) 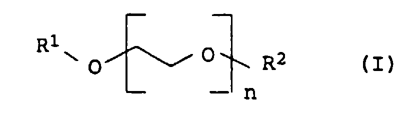 dans laquelle
R¹ et R² représentent un atome d'hydrogène, un groupement alkyle en C₁-C₂₀, aryle, 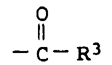 ou bien R¹ et R² représentent ensemble une chaîne alkylène en C₀-C₂₀,
R³ représente un atome d'hydrogène, un groupement alkyle en C₁ -C₂₀ ou aryle et
n vaut 0 à 30,
étant entendu que R¹ et R² ne représentent pas 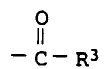 lorsque n est mis pour 0 ou 1, et que R¹ et R² ne représentent pas des atomes d'hydrogène lorsque n est mis pour 0.

7. Procédé de préparation d'acides carboxyliques à partir d'oléfines et de monoxyde de carbone selon l'une quelconque des revendications 1 à 4, caractérisé en ce que l'on effectue la réaction dans l'acide carboxylique aqueux à 10-90% en poids, en tant que solvant.

8. Procédé de préparation d'acides carboxyliques à partir d'oléfines et de monoxyde de carbone selon l'une quelconque des revendications 1 à 6, caractérisé en ce que l'on utilise du monoxyde de carbone et l'oléfine dans un rapport en moles de 0,9 : 1 à 20 : 1.

9. Procédé de préparation d'acides carboxyliques à partir d'oléfines et de monoxyde de carbone selon l'une quelconque des revendications 1 à 7, caractérisé en ce que l'on utilise de l'éthylène en tant qu'oléfine.

10. Procédé de préparation d'acides carboxyliques à partir d'oléfines et de monoxyde de carbone selon l'une quelconque des revendications 1 à 8, caractérisé en ce que l'on utilise du monoxyde de carbone et de l'éthène dans un rapport en moles de 0,9 : 1 à 1,2 : 1.

11. Procédé de préparation d'acides carboxyliques à partir d'oléfines et de monoxyde de carbone selon l'une quelconque des revendications 1 à 9, caractérisé en ce que l'on effectue la réaction en l'absence d'un hydracide halogéné ou d'un promoteur d'halogène.
